(19) Europäisches Patentamt
European Patent Office
Office européen des brevets

(11) Numéro de publication : **0 176 398**
**B1**

(12) **FASCICULE DE BREVET EUROPÉEN**

(45) Date de publication du fascicule du brevet : **20.09.89**

(51) Int. Cl.$^4$ : **C 07 D245/00**

(21) Numéro de dépôt : **85401671.4**

(22) Date de dépôt : **22.08.85**

(54) **Procédé d'amination de diènes conjugués.**

(30) Priorité : **23.08.84 FR 8413127**

(43) Date de publication de la demande : **02.04.86 Bulletin 86/14**

(45) Mention de la délivrance du brevet : **20.09.89 Bulletin 89/38**

(84) Etats contractants désignés : **AT BE CH DE FR GB IT LI LU NL SE**

(56) Documents cités :
**US-A- 4 104 471**
**US-A- 4 219 677**
**US-A- 4 260 750**
**Le dossier contient des informations techniques présentées postérieurement au dépôt de la demande et ne figurant pas dans le présent fascicule.**

(73) Titulaire : **RHONE-POULENC SANTE**
**20, avenue Raymond Aron**
**F-92160 Antony (FR)**

(72) Inventeur : **Mignani, Gérard**
**2 avenue des Frères Lumière**
**F-69008 Lyon (FR)**
Inventeur : **Morel, Didier**
**3 rue Jean Jaurès**
**F-91700 Villiers sur Orge (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 176 398 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé d'addition sélective d'une amine secondaire cyclique sur un diène conjugué.

Plus particulièrement l'invention concerne l'addition sélective d'une amine secondaire sur l'atome de carbone 4 d'un butadiène-1,3 portant un substituant sur l'atome de carbone 2.

Il est connu de faire réagir des amines secondaires sur des diènes, tel que l'isoprène ou le butadiène en présence de catalyseurs choisis parmi les complexes du palladium, du nickel, du cobalt, du rhodium ou de l'iridium, éventuellement en présence de ligands tels que les phosphines [R. BAKER, Chemical Reviews 73 (5) 505 (1973) ; R. BAKER et Coll., J.C.S. PERKIN II, 1511 (1974) ; R. BAKER et Coll., J.C.S. PERKIN II, 1133 (1975), M. HIDAI et Coll., Bull. Soc. Chim. Japan, 53 2091 (1980)]. La mise en œuvre de ces réactions conduit généralement à des mélanges complexes contenant essentiellement des dérivés dialcoylaminés de l'octadiène-2,6.

Il est connu également de préparer la N,N-diéthylgéranylamine par action de la diéthylamine sur le myrcène en présence de sodium métallique [K. TAKABE et Coll., Bull. Chem. Soc. Japan, 46, 222 (1973)] et la N,N-diméthyl méthyl-2 butène-2 ylamine par action de la diméthylamine sur l'isoprène en présence de butyllithium [G.K. NOREN, J. Org. Chem., 40 (7) 967 (1975) ; K. TAKABE et Coll., Chemistry Letters, 1031 (1975)].

Dans le brevet américain US 4 219 677 est décrit un procédé de télomérisation des oléfines par traitement d'un diène conjugué tel que le butadiène, par un composé contenant un atome d'hydrogène mobile, tel qu'une amine secondaire, en présence d'un catalyseur métallique et d'une phosphine sulfonée.

Dans le brevet européen EP 0 044 771 est décrit un procédé d'addition d'un composé à groupement méthylène actif sur un diène conjugué terminal en présence d'un catalyseur constitué d'un dérivé du rhodium et d'une phosphine soluble dans l'eau.

Compte tenu des différences de comportement entre un composé à groupement méthylène actif et une amine secondaire, l'homme de l'art n'était pas conduit à appliquer à une amine secondaire les conditions de l'addition d'un composé à groupement méthylène actif. En effet, d'après J. MARCH, Advanced Organic Chemistry, 2nd edition, Mc. Graw Hill, p. 228 - 229, les composés à groupement méthylène actif ont un pKa voisin de 11 (liaison C-H) qui est très différent de celui d'une amine secondaire qui est voisin de 30 (liaison N-H).

— d'après JODOGNE et DESSART, Chimie, Ed. A. de BOECK, l'électronégativité d'un atome de carbone est voisine de 2,5 unités alors que celle d'atome d'azote est voisine de 3 unités.

— il y a une délocalisation de l'anion d'un composé à groupement méthylène actif, et en particulier d'un β-cétoester qui n'existe pas avec celui d'une amine secondaire. Par ailleurs, avec une amine secondaire, il n'y a pas formation d'amidure dans l'eau.

Les β-cétoesters et les amines secondaires s'additionnent sur les diènes conjugués terminaux en faisant appel à des mécanismes différents qui n'appellent pas la transposition d'un procédé de l'un à l'autre.

Il a maintenant été trouvé, et c'est ce qui fait l'objet de la présente invention, un procédé d'addition sélective d'une amine secondaire cyclique de formule générale :

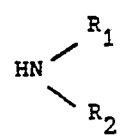

(I)

dans laquelle $R_1$ et $R_2$ forment ensemble avec l'atome d'azote auquel ils sont liés un radical pipéridino ou morpholino, sur un butadiène-1,3 de formule générale :

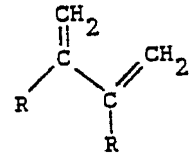

(II)

dans laquelle les symboles R, identiques ou différents, représentent un atome d'hydrogène ou un radical hydrocarboné aliphatique choisi parmi les radicaux alcoyles contenant 1 à 20 atomes de carbone et les radicaux alcényles contenant 2 à 20 atomes de carbone et contenant une ou plusieurs doubles liaisons, dans l'eau ou dans un milieu hydroalcoolique contenant au maximum 50 % en volumes d'un alcool

aliphatique contenant 1 à 3 atomes de carbone en présence d'un catalyseur constitué : d'une part, par au moins une phosphine soluble dans l'eau et, d'autre part, par au moins un composé du rhodium, le catalyseur étant en solution dans l'eau.

D'un intérêt particulier sont les produits de formule générale (II) dans laquelle un des symboles R représente un radical $—CH_3$ (isoprène), $—C_6H_{11}$ (myrcène) ou $—C_{11}H_{19}$ (β-farnésène) et l'autre représente un atome d'hydrogène.

La mise en œuvre du procédé selon l'invention conduit généralement à la formation d'un mélange des produits de formules générales :

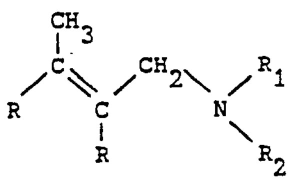

et

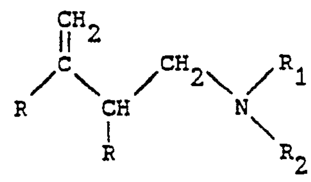

(IIIa) (IIIb)

dans lesquelles R, $R_1$, et $R_2$ sont définis comme précédemment, et également de l'énamine de formule générale :

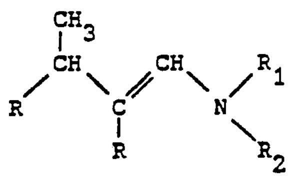

(IV)

dans laquelle R, $R_1$ et $R_2$ sont définis comme précédemment.

Quoi qu'il en soit, le procédé selon la présente invention permet une très grande sélectivité de l'attaque de l'amine secondaire de formule générale (I) sur le diène de formule générale (II).

Les phosphines solubles dans l'eau utilisables dans le cadre de la présente invention sont celles décrites dans le brevet français FR 2 366 237.

Plus particulièrement, on préfère utiliser au moins une phosphine de formule :

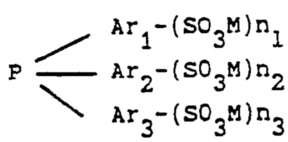

(V)

dans laquelle :

$Ar_1$, $Ar_2$ et $Ar_3$ identiques ou différents représentent chacun un radical choisi parmi le groupe comprenant les radicaux phénylène et les radicaux naphtylène, ces radicaux étant éventuellement substitués.

M est un reste cationique d'origine minérale ou organique choisi de manière que la phosphine de formule (V) soit soluble dans l'eau.

$n_1$, $n_2$ et $n_3$ identiques ou différents sont des nombres entiers supérieurs ou égaux à 0 et inférieurs ou égaux à 3, l'un au moins étant supérieur ou égal à 1.

Les radicaux phénylène et naphtylène peuvent être substitués par tous radicaux ne contrariant pas la solubilité de la phosphine (V) dans l'eau. Parmi ceux-ci, on peut citer, à titre d'exemples, les radicaux alkyle ayant 1 à 6 atomes de carbone, les radicaux alkoxy ayant de 1 à 6 atomes de carbone, les atomes d'halogène, les radicaux —OH, —CN, $—NO_2$, $—N\text{-(alkyl)}_2$, caboxylates.

Le procédé selon l'invention est de préférence mis en œuvre en utilisant au moins une phosphine de formule (V) dans laquelle $Ar_1$, $Ar_2$ et $Ar_3$ identiques ou différents représentent chacun un radical choisi parmi le groupe comprenant le radical phénylène et les radicaux phénylène substitués.

Encore plus préférentiellement, on utilise une phosphine dans laquelle au moins un des groupements $SO_3M$ est en position méta sur le noyau benzénique.

De préférence, M est choisi parmi le groupe comprenant les cations dérivés des métaux Na, K, Ca, Ba, les ions $NH_4^+$ et ammonium quaternaire comme les ions tétraméthylammonium, tétrapropylammonium et tétrabutylammonium.

$n_1$, $n_2$ et $n_3$ sont de préférence égaux à 0 ou 1, $n_1 + n_2 + n_3$ étant compris entre 1 et 3 ($1 < n_1 + n_2 + n_3 < 3$.).

Les composés de formule (V) plus particulièrement préférés sont les phosphines de formules suivantes :

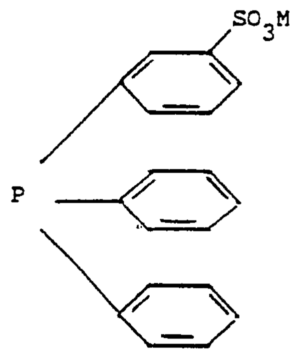 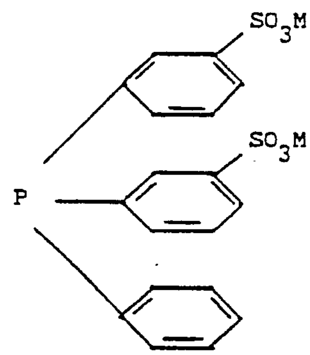 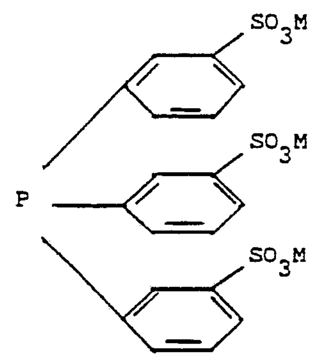

où M a la signification précédente.

Le composé du rhodium utilisé doit être soluble dans l'eau ou capable de passer en solution dans l'eau dans les conditions de la réaction, par réaction de coordination avec les phosphines hydrosolubles. Le reste lié au métal n'est pas critique dès lors qu'il satisfait à ces conditions.

Selon un mode de réalisation préférentiel du procédé le dérivé du rhodium est choisi parmi le groupe comprenant les sels inorganiques, organiques et les complexes du rhodium comme par exemple $RhCl_3$, $RhBr_3$, $Rh_2O$, $Rh_2O_3$, $Rh(NO_3)_3$, $Rh_2(CH_3COO)_4$, $Rh(CH_3\ COCHCOCH_3)_3$, $[RhCl(\text{cyclooctadiène-1,5})]_2$, $[RhCl(CO)_2]_2$, $RhCl_3\ (C_2H_5NH_2)_3$.

On préfère tout particulièrement utiliser $RhCl_3$ et $[RhCl(\text{cyclooctadiène-1,5})]_2$.

On utilise une quantité de rhodium ou de composé du rhodium telle que le nombre d'atomes-gramme de rhodium élémentaire par litre de solution réactionnelle soit compris entre $10^{-4}$ et 1. De préférence, il est compris entre 0,001 et 0,5.

Pour une bonne mise en œuvre du procédé, la quantité de phosphine est choisie de telle sorte que le nombre d'atomes-gramme de phosphore trivalent rapporté à un atome-gramme de rhodium soit compris entre 0,1 et 200. De préférence, ce nombre est compris entre 3 et 100.

La température à laquelle est conduite la réaction peut varier dans de larges limites. Préférentiellement, on opère à des températures modérées inférieures à 200 °C. Plus particulièrement la température est comprise entre 50 °C et 125 °C.

Le rapport molaire du composé I au composé II n'est pas critique. On préfère opérer avec un léger excès molaire de composé I.

La quantité d'eau minimum nécessaire est celle suffisante pour dissoudre le catalyseur en totalité et au moins une partie des réactifs I et II, la réaction ayant lieu en phase aqueuse, les produits de la réaction étant en phase organique non miscible à l'eau.

Afin d'augmenter la cinétique de la réaction et de faciliter le recyclage du catalyseur, il est possible d'opérer en présence d'un co-solvant. Plus particulièrement une partie de l'eau nécessaire à la mise en œuvre de la réaction est remplacée par une quantité équivalente d'un alcool aliphatique contenant 1 à 3 atomes de carbone, tel que le méthanol, l'éthanol ou l'isopropanol. La quantité d'eau qui peut être remplacée est au maximum égale à la moitié de la quantité d'eau nécessaire à la mise en œuvre de la réaction sans co-solvant.

Une manière pratique de mettre en œuvre le procédé de l'invention consiste à charger dans un réacteur convenable après l'avoir purgé à l'aide d'un gaz inerte (azote, argon) soit la solution aqueuse ou hydroalcoolique catalytique préalablement formée, soit les divers composants : phosphine, eau, éventuellement l'alcool tel que le méthanol, l'éthanol ou l'isopropanol, composé du rhodium. On porte le réacteur à la température de réaction avant ou après l'introduction de l'amine secondaire de formule générale (I) qui elle-même peut être introduite avant, après ou simultanément au butadiène substitué.

Après arrêt de la réaction, on refroidit à la température ambiante. Le contenu du réacteur est soutiré et on isole ensuite le produit de la réaction qui est en phase organique en séparant cette dernière de la phase aqueuse contenant le catalyseur par décantation et éventuellement par une extraction à l'aide d'un solvant convenable.

La solution aqueuse ou hydroalcoolique résiduelle peut être recyclée dans le réacteur pour catalyser une nouvelle réaction. En particulier, en solution hydro-méthanolique le catalyseur peut être recyclé plus de 25 fois sans perte d'activité. La solution aqueuse ou hydroalcoolique peut aussi rester dans le réacteur, les produits organiques étant alors soutirés après décantation.

Le procédé selon l'invention permet d'obtenir des sélectivités voisines de 100 % en composés (III a), (III b) et (IV). Ces composés peuvent être séparés par tous moyens connus de l'homme de l'art, comme par exemple par distillation.

Les produits de formule générale (III b) obtenus selon le procédé de la présente invention peuvent, en opérant en particulier dans les conditions du brevet français FR 7 007 199 publié sous le numéro 2 031 335, être isomérisés en produit de formule générale (III a). Généralement cette isomérisation peut s'effectuer en opérant à une température voisine de 25 °C en présence d'un catalyseur tel que le palladium sur charbon.

Les produits de formule générale (III a), (III b) et (IV) obtenus selon le procédé de la présente invention sont particulièrement utiles comme intermédiaires en synthèse organique pour la préparation de vitamines et de parfums.

Par exemple, les produits de formule générale (III a), (III b) et (IV) dans lesquelles R représente un radical $—C_6H_{11}$ sont utiles pour préparer le citral (diméthyl-3,7 octadiène-2,6 al) ou l'hydroxycitronellal (hydroxy-7 diméthyl-3,7 octanal) selon le procédé décrit par K. TAKABE et Coll., Synthetic Communications, 13, (4) 297 (1981) ; K. TAKABE et Coll., Chemistry letters, 1987 (1982) et brevets américains US 3 932 539, US 4 266 087.

Les exemples suivants, donnés à titre non limitatif, montrent comment l'invention peut être mise en pratique.

Exemple 1

Dans un autoclave en acier inoxydable de 125 $cm^3$, on introduit, sous atmosphère d'argon, 0,101 g de $[RhCl(cyclooctadiène-1,5)]_2$ soit 0,4097 milli-atome-gramme de rhodium, 1,287 g de :

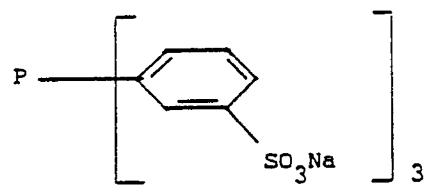

(qui sera désigné par la suite TPPTS Na) soit 2,1 milli-atome-gramme de $P^{3+}$, 15 $cm^3$ d'eau dégazée, 13,33 g de morpholine (0,153 mole) et 10,62 g d'isoprène (0,156 mole). On laisse réagir pendant 16 heures 30 minutes à 100 °C. Après refroidissement, le mélange réactionnel est séparé, par décantation, en deux phases : une phase aqueuse orangée (19,8 g) et une phase organique jaune (19,7 g).

La phase organique est concentrée sous pression réduite. On obtient ainsi 16,74 g d'une huile légèrement jaune. Par distillation sous pression réduite (1 mm de mercure ; 0,13 kPa) à 41-43 °C, on obtient 11,4 g d'un mélange constitué, d'après l'analyse par résonance magnétique nucléaire, de :

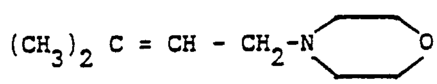 (38 %)

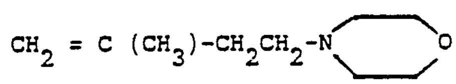 (54 %)

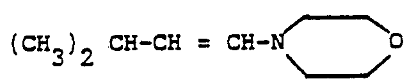 (8 %)

Un dosage par chromatographie en phase gazeuse avec un étalon interne, du produit brut de la réaction montre que le taux de transformation de l'isoprène est de 72 % et que le rendement en produits aminés est de 65 %. La structure des produits obtenus est confirmée par le spectre infra-rouge, le spectre de masse et le spectre de résonance magnétique nucléaire du proton.

Exemple 2

Dans un autoclave en acier inoxydable de 125 $cm^3$, on introduit, sous atmosphère d'argon, 0,1021 g de $[RhCl(cyclooctadiène-1,5)]_2$ soit 0,4136 milli-atome-gramme de rhodium, 1,2913 g de TPPTS Na soit 2,10 milli-atome-gramme de $P^{+3}$, 14,82 g d'eau dégazée, 13,46 g de morpholine (0,1545 mole) et 21,30 g de myrcène (0,1566 mole). On chauffe pendant 21 heures 30 minutes à 100 °C. Le mélange réactionnel est traité dans les conditions décrites dans l'exemple 1. Après évaporation de la phase organique, on obtient 29,56 g d'huile constituée, d'après l'analyse par résonance magnétique nucléaire, de :

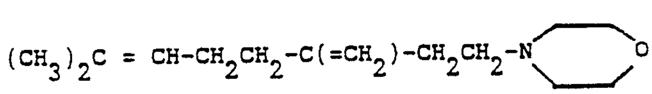 (53 %)

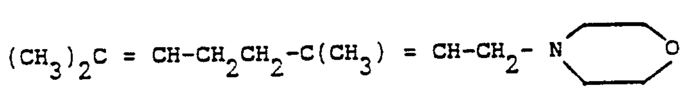 (31 %)

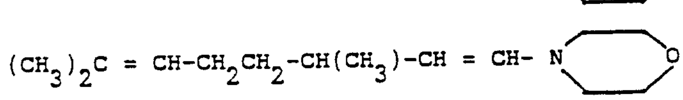 (16 %)

Un dosage, par chromatographie en phase gazeuse avec étalon interne, du produit brut de la réaction montre que le taux de transformation du myrcène est de 76 % et que le rendement en produits aminés est de 59 %.

La structure des produits obtenus est confirmée par le spectre infra-rouge, le spectre de masse et le spectre de résonance magnétique nucléaire du proton.

Exemple 3

Dans un autoclave en acier inoxydable de 125 cm$^3$, on introduit sous atmosphère d'argon, 0,1008 g de $[RhCl(cyclooctadiène\text{-}1,5)]_2$ soit 0,409 milli-atome-gramme de rhodium, 1,2908 g de TPPTS Na soit 2,1 milli-atome-gramme de $P^{3+}$, 14,88 g d'eau distillée, 12,27 g de pipéridine (0,1511 mole) et 10,69 g d'isoprène (0,1572 mole). On chauffe pendant 21 heures à 100 °C. Le mélange réactionnel est traité dans les conditions décrites dans l'exemple 1. Après évaporation de la phase organique (20,4 g), on obtient 7,17 g d'une huile légèrement constituée, d'après l'analyse par résonance magnétique nucléaire, de :

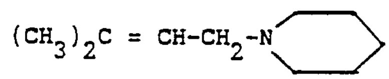 (33 %)

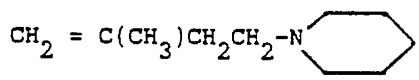 (29 %)

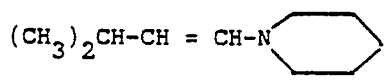 (38 %)

Un dosage par chromatographie en phase gazeuse avec étalon interne du produit brut de la réaction montre que le rendement en produits aminés est de 27,4 %.

La structure des produits obtenus est confirmée par le spectre infra-rouge, le spectre de masse et le spectre de résonance magnétique nucléaire du proton.

Exemple 4

Dans un autoclave en acier inoxydable de 125 cm$^3$, on introduit, sous atmosphère d'argon, 0,1014 g de $[RhCl(cyclooctadiène\text{-}1,5)]_2$ soit 0,411 milli-atome-gramme de rhodium, 1,2939 g de TPPTS Na soit 2,11 milli-atome-gramme de $P^{+3}$, 14,95 g d'eau, 6,77 g de morpholine (0,0771 mole) et 21,65 g de myrcène (0,1592 mole). On chauffe pendant 21 heures 25 minutes à 100 °C. Le mélange réactionnel est traité dans les conditions décrites dans l'exemple 1.

On obtient 27,14 g d'une phase organique qui contient un mélange des dérivés aminés suivants :

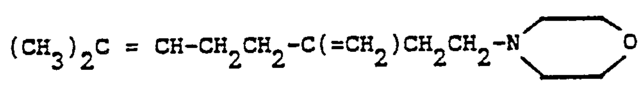 (54 %)

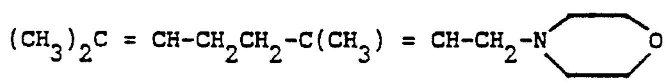 (30 %)

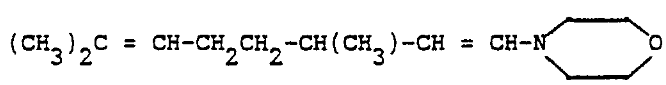 (16 %)

Un dosage par chromatographie en phase gazeuse avec étalon interne montre que le rendement en produits aminés est de 81,2 % par rapport à la morpholine mise en œuvre.

Exemple 5

Dans un autoclave en acier inoxydable de 125 m$^3$, on introduit sous atmosphère d'argon, 0,1012 g de $[RhCl(cyclooctadiène\text{-}1,5)]_2$ soit 0,410 milli-atome-gramme de rhodium, 1,2902 g de TPPTS Na soit 2,10 milli-atome-gramme de $P^{+3}$, 14,87 g d'eau, 21,57 g de myrcène (0,1586 mole) et 12,93 de pipéridine (0,1518 mole). On chauffe pendant 20 heures 15 minutes à 100 °C.

Le mélange réactionnel est traité dans les conditions décrites dans l'exemple 1.

On obtient 30,46 g d'une phase organique qui, après concentration, fournit 29,14 g d'une huile légèrement jaune qui contient les dérivés aminés suivants :

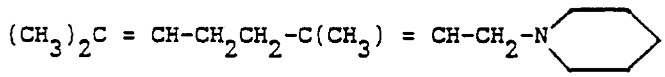 (31 %)

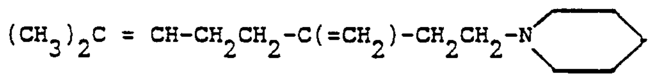 (31 %)

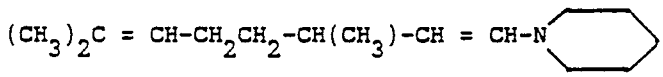 (38 %)

La structure des produits obtenus est confirmée par le spectre infra-rouge, le spectre de masse et le spectre de résonance magnétique nucléaire du proton.

Exemple 6

Dans un autoclave en acier inoxydable de 125 $cm^3$, on introduit sous atmosphère d'argon, 0,0617 g de $[RhCl(cyclooctadiène\text{-}1,5)]_2$ soit 0,25 milli-atome-gramme de rhodium, 0,74 g de TPPTS Na soit 1,21 milli-atome-gramme de $P^{+3}$, 9,24 g d'eau, 7,61 g de morpholine (0,08735 mole) et 7,37 g de diméthyl-2,3 butadiène-1,3 (0,08971 mole). On chauffe pendant 10 heures 35 minutes à 100 °C. Le mélange réactionnel est traité dans les conditions décrites dans l'exemple 1. On obtient une phase organique (7,56 g) qui, après concentration, fournit 1,16 g d'un produit jaune huileux constitué de :

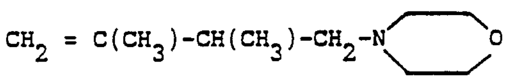 (30 %)

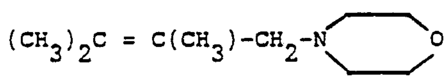 (32 %)

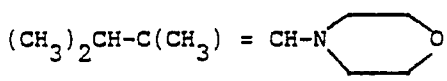 (38 %)

La structure des produits obtenus est confirmée par le spectre infra-rouge, le spectre de masse et le spectre de résonance magnétique nucléaire du proton.

Exemple 7

Dans un autoclave en acier inoxydable de 125 $cm^3$, on introduit sous atmosphère d'argon 0,1229 g de $[RhCl(cyclooctadiène\text{-}1,5)]_2$ soit 0,497 milli-atome-gramme de rhodium, 1,5557 g de TPPTS Na soit 2,54 milli-atome-gramme de $P^{+3}$, 17,9 g d'eau, 16,05 g de morpholine (0,1842 mole) et 10 g de butadiène (0,1852 mole). On chauffe pendant 20 heures à 100 °C. Le mélange réactionnel est traité dans les conditions décrites dans l'exemple 1. On obtient 13,49 g d'une phase organique contenant :

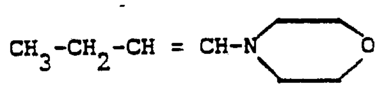

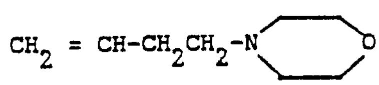

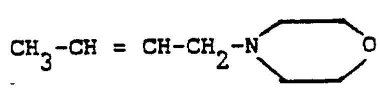

## Revendications

1. Procédé d'addition sélective d'une amine secondaire cyclique de formule générale :

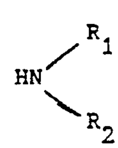

dans laquelle $R_1$ et $R_2$ forment ensemble avec l'atome d'azote auquel ils sont liés un radical pipéridino ou morpholino, sur un butadiène-1,3 de formule générale :

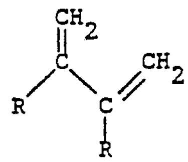

dans laquelle les symboles R, identiques ou différents, représentent un atome d'hydrogène ou un radical hydrocarboné aliphatique choisi parmi les radicaux alcoyles contenant 1 à 20 atomes de carbone et les radicaux alcényles contenant 2 à 20 atomes de carbone et contenant une ou plusieurs doubles liaisons caractérisé en ce que l'on opère dans l'eau ou dans un milieu hydroalcoolique contenant au maximum 50 % en volumes d'un alcool aliphatique contenant 1 à 3 atomes de carbone en présence d'un catalyseur soluble dans l'eau, constitué, d'une part, par une phosphine sulfonée soluble dans l'eau et, d'autre part, par un composé du rhodium, et isole le mélange des produits de formule générale :

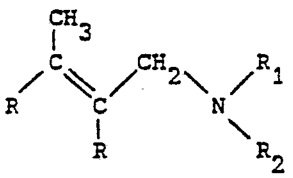

,

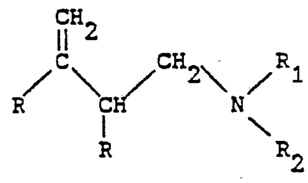

et

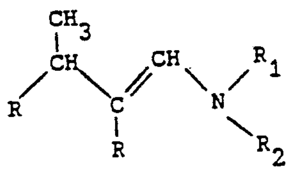

dans laquelle les symboles R, $R_1$ et $R_2$ sont définis comme précédemment.

2. Procédé selon la revendication 1 caractérisé en ce que le composé du rhodium est choisi parmi $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(cyclooctadiène-1,5)]_2$, $[RhCl(CO)_2]_2$ et $RhCl_3(C_2H_5NH_2)_3$.

3. Procédé selon la revendication 1 caractérisé en ce que le composé du rhodium est $[RhCl(cyclooctadiène-1,5)]_2$.

4. Procédé selon la revendication 1 caractérisé en ce que la phosphine sulfonée est la tri-(métasulfophényl)phosphine.

5. Procédé selon l'une des revendications 1 à 3 caractérisé en ce que l'on met en œuvre une quantité de rhodium telle que le nombre d'atomes-gramme de rhodium élémentaire par litre de solution réactionnelle est compris entre $10^{-4}$ et 1.

6. Procédé selon la revendication 4 caractérisé en ce que le nombre d'atomes-gramme de rhodium par litre de solution réactionnelle est compris entre 0,001 et 0,5.

7. Procédé selon l'une des revendications 1 à 6 caractérisé en ce que la quantité de phosphine soluble dans l'eau est telle que le nombre d'atomes-gramme de phosphore trivalent rapporté à un atome-gramme de rhodium est compris entre 0,1 et 200.

8. Procédé selon la revendication 7 caractérisé en ce que le nombre d'atomes-gramme de phosphore trivalent rapporté à un atome-gramme de rhodium est compris entre 3 et 100.

9. Procédé selon la revendication 1 caractérisé en ce que le dérivé du butadiène-1,3 est choisi parmi l'isoprène, le myrcène et le β-farnésène.

10. Procédé selon l'une des revendications 1 à 9 caractérisé en ce que l'on opère à une température inférieure à 200 °C.

## Claims

1. A process for selective addition of a cyclic secondary amine of general formula :

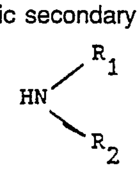

in which $R_1$ and $R_2$ form together with the nitrogen atom to which they are attached a piperidino or phorpholino radical, to a 1,3-butadiene of general formula :

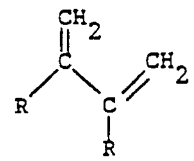

in which the symbols R, which may be identical or different, denote a hydrogen atom or an aliphatic hydrocarbon radical chosen from alkyl radicals containing 1 to 20 carbon atoms and alkenyl radicals containing 2 to 20 carbon atoms, and containing one or more double bonds, wherein the reaction is performed in water or in an aqueous alcoholic medium containing at most 50 % by volume of an aliphatic alcohol containing 1 to 3 carbon atoms, in the presence of a water-soluble catalyst consisting of, on the one hand a water-soluble sulphonated phosphine, and on the other hand a rhodium compound, and the mixture of the products of general formula :

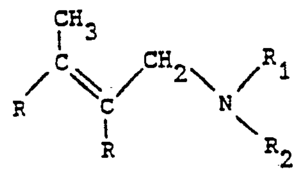

,

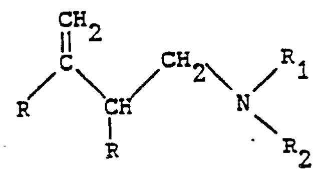

and

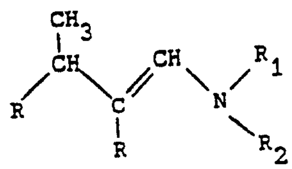

in which the symbols R, $R_1$ and $R_2$ are defined as above, is isolated.

2. A process according to claim 1, wherein the rhodium compound is chosen from $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, $[RhCl(1,5\text{-cyclooctadiene})]_2$, $[RhCl(CO)_2]_2$ and $RhCl_3(C_2H_5NH_2)_3$.

3. A process according to claim 1, wherein the rhodium compound is $[RhCl(1,5\text{-cyclooctadiene})]_2$.

4. A process according to claim 1, wherein the sulphonated phosphine is tri(meta-sulphophenyl)phosphine.

5. A process according to one of claims 1 to 3, wherein an amount of rhodium is used such that the number of gram-atoms of elementary rhodium per litre of reaction solution is between $10^{-4}$ and 1.

6. A process according to claim 4, wherein the number of gram-atoms of rhodium per litre of reaction solution is between 0.001 and 0.5.

7. A process according to one of claims 1 to 6, wherein the amount of water-soluble phosphine is such that the number of gram-atoms of trivalent phosphorus relative to one gram-atom of rhodium is between 0.1 and 200.

8. A process according to claim 7, wherein the number of gram-atoms of trivalent phosphorus relative to one gram-atom of rhodium is between 3 and 100.

9. A process according to claim 1, wherein the 1,3-butadiene derivative is chosen from isoprene, myrcene and β-farnesene.

10. A process according to one of claims 1 to 9, wherein the reaction is performed at a temperature below 200 °C.

**Patentansprüche**

1. Verfahren zur selektiven Addition eines cyclischen sekundären Amins der allgemeinen Formel :

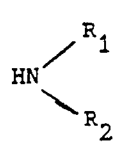

in welcher $R_1$ und $R_2$ zusammen mit dem Stickstoffatom, an das sie gebunden sind, einen Piperidino- oder Morpholinorest bilden, an ein 1,3-Butadien der allgemeinen Formel :

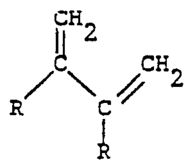

in welcher die Symbole R unabhängig voneinander ein Wasserstoffatom oder einen aliphatischen Kohlenwasserstoffrest, ausgewählt aus den Alkylresten mit 1 bis 20 Kohlenstoffatomen und den Alkenylresten mit 2 bis 20 Kohlenstoffatomen, die eine oder mehrere Doppelbindungen enthalten, darstellen, dadurch gekennzeichnet, daß man in Wasser oder in einem Wässerig-alkoholischen Milieu, das höchstens 50 Vol.-% eines aliphatischen Alkohols mit 1 bis 3 Kohlenstoffatomen enthält, in Gegenwart eines in Wasser löslichen Katalysators arbeitet, der einerseits aus einem in Wasser löslichen sulfonierten Phosphin und anderseits aus einer Rhodiumverbindung besteht, und die Mischung von Verbindungen der allgemeinen Formel :

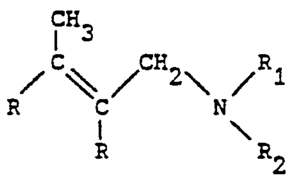

,

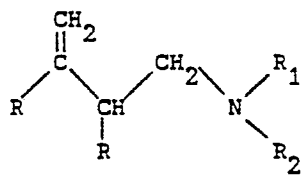

und

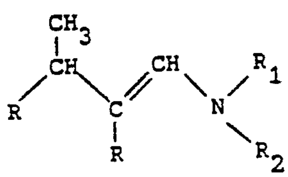

in welcher die Symbole R, $R_1$ und $R_2$ die obige Bedeutung haben, isoliert.

2. Verfahren nach Anspruch 1, dadurch gekennzeichnet, daß die Rhodiumverbindung ausgewählt ist aus $RhCl_3$, $RhBr_3$, $Rh_2O$, $Rh_2O_3$, $Rh(NO_3)_3$, $Rh_2(CH_3COO)_4$, $Rh(CH_3COCHCOCH_3)_3$, [RhCl(1,5-cyclooctadien)]$_2$, $[RhCl(CO)_2]_2$ und $RhCl_3(C_2H_5NH_2)_3$.

3. Verfahren nach Anspruch 1, dadurch gekennzeichnet, daß die Rhodiumverbindung [RhCl(1,5-cyclooctadien)]$_2$ ist.

4. Verfahren nach Anspruch 1, dadurch gekennzeichnet, daß das sulfonierte Phosphin Tri-(metasulfophenyl)phosphin ist.

5. Verfahren nach einem der Ansprüche 1 bis 3, dadurch gekennzeichnet, daß man eine solche Menge an Rhodium einsetzt, daß die Anzahl an Grammatom elementaren Rhodiums pro Liter Reaktionslösung zwischen $10^{-4}$ und 1 liegt.

6. Verfahren nach Anspruch 4, dadurch gekennzeichnet, daß die Anzahl an Grammatom Rhodium pro Liter Reaktionslösung zwischen 0,001 und 0,5 liegt.

7. Verfahren nach einem der Ansprüche 1 bis 6, dadurch gekennzeichnet, daß die Menge an in Wasser löslichem Phosphin derart ist, daß die Anzahl an Grammatom dreiwertigen Phosphors, bezogen auf ein Grammatom Rhodium, zwischen 0,1 und 200 liegt.

8. Verfahren nach Anspruch 7, dadurch gekennzeichnet, daß die Anzahl an Grammatom dreiwertigen Phosphors, bezogen auf ein Grammatom Rhodium, zwischen 3 und 100 liegt.

9. Verfahren nach Anspruch 1, dadurch gekennzeichnet, daß das 1,3-Butadienderivat ausgewählt ist aus Isopren, Myrcen und β-Farnesen.

10. Verfahren nach einem der Ansprüche 1 bis 9, dadurch gekennzeichnet, daß man bei einer Temperatur unterhalb 200 °C arbeitet.